# EUROPEAN PATENT SPECIFICATION

(11) **EP 4 465 907 B1**
(45) Date of publication and mention of the grant of the patent: **17.09.2025**
(21) Application number: 22701349.7
(22) Date of filing: 17.01.2022
(51) Int. Cl.: A61B 17/32, A61B 17/3207, A61B 18/14, A61M 1/36

(54) **A SYSTEM FOR FORMING A FISTULA**
SYSTEM ZUR BILDUNG EINER FISTEL
UN SYSTÈME POUR FORMER UNE FISTULE

(43) Date of publication of application: 27.11.2024
(73) Proprietor: Clearstream Technologies Limited, Enniscorthy, County Wexford (IE)
(72) Inventor: WHELAN, Michael, Enniscorthy, County Wexford (IE); BROADERS, Nicola, Enniscorthy, County Wexford (IE); PALMER, Olivia Ruth, Enniscorthy, County Wexford (IE); O´SHEA, John, Enniscorthy, County Wexford (IE)
(74) Representative: Hoffmann Eitle
(86) International application number: PCT/EP2022/050846
(87) International publication number: WO 2023/134871

(56) References cited:
- WO-A1-2021/108600
- US-A1- 2017 202 616
- US-B1- 9 649 157

## Description

### Technical Field

The present disclosure relates to a system for forming a fistula between two vessels.

### Background

A fistula denotes a passageway formed between two internal organs. Forming a fistula between two blood vessels can have one or more beneficial functions. For example, the formation of a fistula between an artery and a vein may provide access to the vasculature for haemodialysis patients. Specifically, forming a fistula between an artery and a vein allows blood to flow quickly between the vessels while bypassing the capillaries. Needles, catheters, or other cannulas may then be inserted into the blood vessels near the fistula to draw blood from the circulatory system, pass it through a dialysis machine, and return it to the body. The quickened flow provided by the fistula may provide for effective haemodialysis.

In other instances, a fistula may be formed between two veins to form a veno-venous fistula. Such a veno-venous fistula may be used to treat portal venous hypertension. Specifically, cirrhosis or other liver diseases may cause increased resistance to flow from the portal veins draining from the intestine to the liver. This increased resistance may cause massive dilation of blood vessels, which may rupture spontaneously. To help prevent this undesirable outcome, a fistula may be formed between a portal vein and one of the major branches, thereby lowering venous pressure in the portal vein

US 2012/0302935 A1 and US 2017/0202616 A1 disclose systems and methods for forming a fistula using an endovascular approach. These systems comprise a first catheter having a housing with an electrode and a second catheter having a housing with a backstop. However, these systems are limited in calcified vessels by the distance the electrode can fire through the venous/arterial walls. Furthermore, due to the rigid ceramic housing, it can be difficult to navigate these catheters through tortuous vessel anatomy, such as the anterior tibial vessel, for example.

In view of the above, there is a need in the art for a new catheter system which can more effectively form a fistula in calcified vessels.

Furthermore, there is a need in the art for a new catheter system for forming a fistula which can more easily navigate through the vessel anatomy.

WO 2021/108600 A1 discloses systems, apparatus, and methods for treatment of varicocele and associated conditions. The systems, apparatus, and methods can include forming one or more fluid connections or fistulas between blood vessels such as a gonadal vein (e.g., spermatic vein, ovarian vein) and surrounding veins. The systems, apparatus, and methods can include occluding one or more blood vessels such as a gonadal vein (e.g., spermatic vein, ovarian vein). The systems, apparatus and methods relate to flow diverters, replacement valves, etc., e.g., for treatment of varicocele and associated conditions.

US 9649157 B1 discloses systems and methods for creating arteriovenous fistulas.

US 2017/0202616 A1 discloses devices and methods for forming a fistula. Further background art is disclosed in US 6 287 304 B1.

### Summary

According to the present invention, there is provided a system for forming a fistula between two vessels, according to claim 1. The dependent claims define preferred embodiments.

In a first aspect of the present disclosure, there is provided a system for forming a fistula between two vessels, the system comprising a first catheter. The first catheter comprises a housing having a proximal section and a distal section. An electrode is disposed at least partially within the housing. The electrode comprises a distal portion connected to the distal section of the housing, a proximal portion connected to the proximal section of the housing and an intermediate portion therebetween for contacting a vessel wall and forming the fistula. The proximal housing section and the distal housing section are longitudinally moveable with respect to each other to move the electrode between a contracted configuration and an expanded configuration. The proximal housing section and the distal housing section are connected via at least one flexible connection element. The least one flexible connection element comprises a catheter sleeve.

In some embodiments, this may result in a catheter system where the height of the electrode can be adjusted to more effectively form a fistula in a calcified vessel.

In some embodiments, this may also result in a catheter system which is more flexible and can more easily navigate tortuous vessel anatomy.

Throughout this disclosure, the term 'expanded configuration' of the electrode will be used to denote a configuration where the electrode extends radially further from the housing than in a 'contracted configuration'.

Throughout this disclosure, the term 'fistula' is used to denote a connection or passageway between two vessels.

In some embodiments, this may result in a more stable configuration of the first catheter.

In some embodiments, this may result in a more stable configuration of the housing while still maintaining flexibility of the catheter.

The catheter sleeve may be disposed around at least the proximal and distal sections of the housing.

In some embodiments, this may result in a more stable configuration of the housing while still maintaining flexibility of the catheter.

The housing may comprise an opening. The electrode may extend out of the opening.

The catheter sleeve may comprise an opening. The opening of the catheter sleeve may be aligned with the opening of the housing.

The at least one flexible connection element may comprise a ribbon, wire or microspring.

In some embodiments, this may result in a more stable configuration of the housing while still maintaining flexibility of the catheter.

The ribbon, wire or microspring may be insulated.

In some embodiments, this may prevent arcing between the electrode and the ribbon, wire or microspring.

The ribbon, wire or microspring may be fixed to one of the housing sections and longitudinally moveable with respect to the other housing section.

In some embodiments, this may make it easier for a user to move the proximal and distal housing sections with respect to each other to adjust the height of the electrode.

The ribbon, wire or microspring may be fixed to the proximal housing section and longitudinally moveable with respect to the distal housing section.

In some embodiments, this may allow a user to easily manipulate the height of the electrode by pushing or pulling the proximal section of the housing.

The ribbon, wire or microspring may be in contact with the inner walls of the distal housing section.

In some embodiments, this may allow the ribbon, wire or microspring to act as a rail to stabilise the housing of the catheter.

A pull wire may be connected to the distal housing section to allow the distal housing section to be moved longitudinally with respect to the proximal housing section.

In some embodiments, this may allow a user to easily manipulate the height of the electrode.

The proximal housing section and the distal housing section may not be in contact in the contracted configuration

In some embodiments, this provides the first catheter with flexibility as the housing can bend and more easily navigate tortuous vessel anatomy.

The proximal housing section and the distal housing section may be in contact in the expanded configuration.

In some embodiments, this may provide the user with an indication that the expanded configuration has been reached and prevents over-flexing of the electrode.

The proximal housing section and the distal housing section may not be in contact in the contracted and the expanded configuration.

The electrode may be a leaf spring.

In some embodiments, this may allow the electrode to be bent and flexed without breaking.

Throughout this disclosure, the term 'leaf spring' will be used to refer to a flexible curved strip of material which can be bent but will regain its original shape when released.

The electrode may have a convex portion.

In some embodiments, this may result in good fistula formation.

In some embodiments, this may also allow the electrode to be to be easily bent or flexed to increase the height of the electrode.

The electrode may be a ribbon wire.

In some embodiments, this may result in good fistula formation.

A distal end of the electrode may be fixed to the distal section of the housing.

In some embodiments, this may allow better adjustment of the height of the electrode by moving the two housing sections relative to each other.

A proximal end of the electrode may be fixed to the proximal section of the housing.

The housing may be at least partially made from a ceramic material.

In some embodiments, this may result in a housing which can withstand the current and heat of the electrode.

The system may further comprise a second catheter comprising a second housing and a backstop for the electrode.

The first catheter and the second catheter may comprise one or more sets of magnets positioned to align the electrode with the backstop.

In some embodiments, this may allow quick and simple alignment of the electrode and backstop.

A distal set of magnets may be disposed on the second catheter, distally of the second housing.

A proximal set of magnets may be disposed on the second catheter, proximally of the second housing.

A distal set of magnets may be disposed on the first catheter, distally of the first housing.

A proximal set of magnets may be disposed on the first catheter, proximally of the first housing.

The backstop may be a recessed backstop which has a portion shaped complementary to the electrode.

In some embodiments, this may result in improved fistula formation.

The backstop may have a concave portion.

In some embodiments, this may result in improved fistula formation.

The backstop may be non-conductive.

In some embodiments, this may prevent arcing between the electrode and backstop.

The backstop may be at least partly made from a ceramic material.

In some embodiments, this may result in a backstop which can better withstand the heat and plasma generated by the electrode.

The system may further comprise a first handle connected to the proximal end of the first catheter.

In some embodiments, this may allow a user to more easily move the proximal housing with respect to the distal housing and thereby adjust the height of the electrode.

The system may further comprise a radiofrequency generator for supplying radiofrequency energy to the electrode.

### Brief Description of the Drawings

To enable better understanding of the present disclosure, and to show how the same may be carried into effect, reference will now be made, by way of example only, to the accompanying drawings, in which:
FIG. 1 shows an example of a catheter system for forming a fistula.
FIG. 2 shows a side view of a first catheter in a contracted configuration according to the present disclosure.
FIG. 3 shows a side view of the first catheter of FIG. 2 in an expanded configuration.
FIG. 4 shows a side view of an alternative embodiment of a first catheter according to the present disclosure in an expanded configuration.
FIG. 5 shows a cross-sectional front view of the first catheter of FIG. 4.
FIG. 6 shows a method of forming a fistula using the first catheter of FIG. 4 and a second catheter according to the present disclosure.
FIG. 7 shows the first catheter of FIG. 4 disposed in a venous system.
FIG. 8 shows a side view of an alternative embodiment of a first catheter according to the present disclosure in an expanded configuration.

### Detailed Description

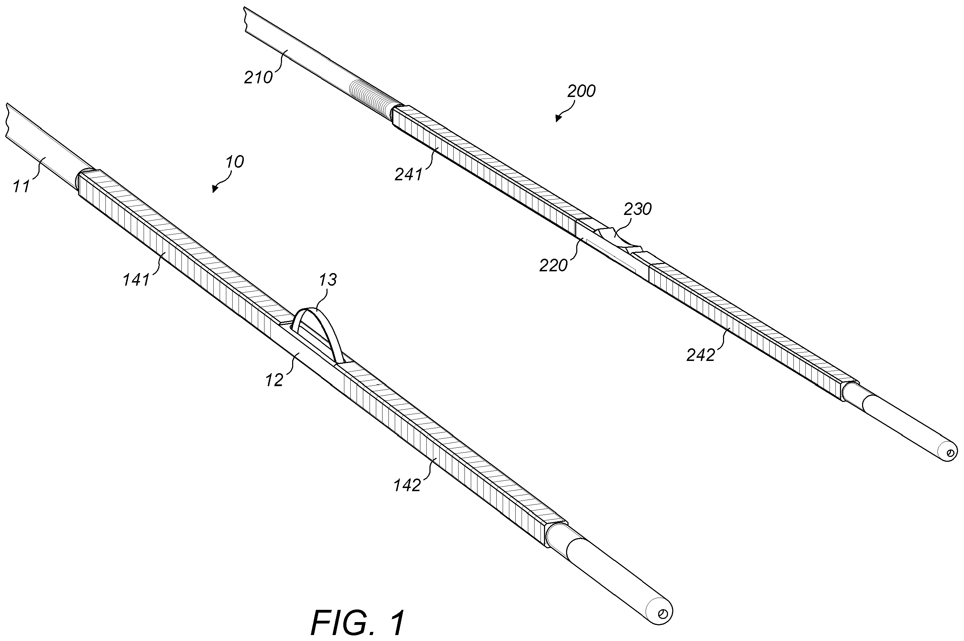

FIG. 1 shows an example of a catheter system for forming a fistula. The system comprises a first catheter 10 and a second catheter 200 which can be used together to form a fistula between two vessels.

The first catheter 10 comprises a catheter shaft 11 and a housing 12 disposed at the distal end of the shaft 11. The housing 12 has an opening and an electrode 13 which is partially disposed in the housing 12 and extends out of the opening. The housing 12 is made from a non-conductive ceramic material which can withstand the heat and plasma generated by the electrode 13. The first catheter 10 also comprises a proximal set of magnets 141 and a distal set of magnets 142 which are disposed proximally and distally of the housing 12, respectively.

The second catheter 200 also comprises a catheter shaft 210 and a second housing 220 having a backstop 230 disposed at the distal end of the shaft 210. The second housing 220 and the backstop 230 are also made from a non-conductive ceramic material to withstand the heat and plasma generated by the electrode. The second catheter 200 also comprises a proximal set of magnets 241, disposed proximally of the housing 220, and a distal set of magnets 242, disposed distally of the housing 220.

In order to form a fistula between two vessels, for example an arteriovenous fistula, the first catheter 10 is introduced into the venous system through an access site and advanced to the treatment site where a fistula is to be formed. The second catheter 200 is introduced into the arterial system through a second access site and also advanced to the treatment site where the fistula is to be formed. The sets of magnets of the first catheter 10 align with the sets of magnets the second catheter 200, resulting in the electrode 13 becoming aligned with the complimentary shaped backstop 230. The backstop 230 is configured to compress the vessel walls in a localised region for ablation by the electrode 13 of the first catheter 10.

Radiofrequency (RF) energy is then supplied to the electrode 13 which allows the electrode 13 to cut through the venous and arterial vessel walls until it hits the backstop 230 to form the fistula.

However, the first catheter is limited in the distance it can cut through the vessel walls. If the vessel walls are calcified, this can substantially increase the thickness of the vessel walls and may make it more difficult to cut through the walls. Furthermore, the ceramic housing 12 of the first catheter 10 is rigid so that in some cases it may be difficult to navigate the first catheter around bends in the vessel anatomy of a patient to reach the treatment site.

The catheter system according to the present disclosure provides a solution to these problems.

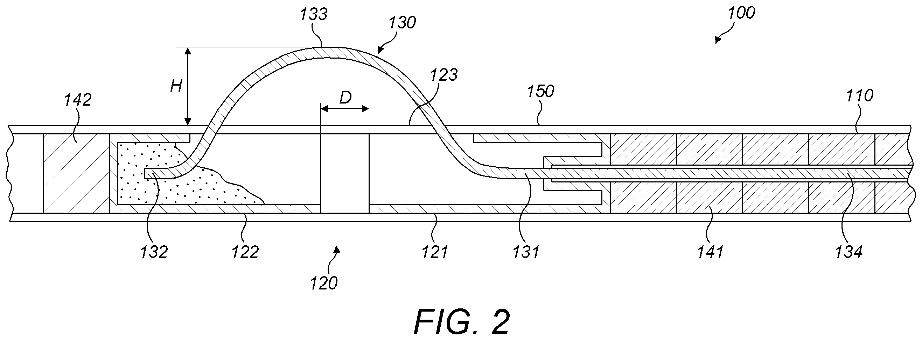

FIG. 2 shows a cross-sectional side view of the distal section of a first catheter 100 according to the present disclosure. This first catheter 100 may be used with a second catheter 200, such as the one described with respect to FIG. 1, to form a fistula. The first catheter 100 comprises a catheter shaft 110 and a housing 120 disposed at the distal end of the catheter shaft 110.

The housing 120 is split into a proximal housing section 121 and a distal housing section 122 and has a lateral opening 123. An electrode 130 is disposed partially within the housing 120. The electrode 130 may be a leaf spring, which may have a proximal portion 131 which is fixed to the proximal housing section 121 and a distal portion 132 which is fixed to the distal housing section 132. The distal portion 132 is shown in FIG. 2 as being fixed to the distal housing section 122 with an adhesive, but may also be fixed with a clamp mechanism, for example. The proximal portion 131 is shown as being fixed to the proximal housing section 121 with a clamp mechanism, but may also be fixed with an adhesive, for example. An intermediate portion 133 of the electrode 130 is positioned between the proximal and the distal portion 131, 132 and extends out of the opening 123 to contact the vessel wall during the fistula formation process. The intermediate portion 133 may have a convex shape which extends away from the housing 120. The distance between the top of the intermediate portion 133 and the housing is the height H of the electrode 130.

The electrode 130 may be in the form of a ribbon wire and may be made from a number of suitable materials, such as one or more refractory metals. For example, the electrode 130 may comprise tungsten, molybdenum, niobium, tantalum, rhenium, or combinations and alloys thereof. The housing 120 may be made from a non-conductive ceramic material which can withstand the heat and plasma generated by the electrode 130. A connecting element 134 may be connected to the proximal portion 131 of the electrode and extends along the length of the catheter shaft 110. The proximal end of the connecting element 134 may then be connected to an RF energy supply, for example in the form of an electrosurgical (ESU) unit, for supplying the electrode 130 with RF energy.

The proximal and distal housing sections 121, 122 are moveable relative to each other. FIG. 2 shows the first catheter 100 with the electrode 130 in a contracted configuration where the height H of the electrode 120 is smaller than in an expanded configuration. In the contracted configuration, the proximal housing section 121 and the distal housing section 122 are not in contact and are separated by a distance D. The proximal and distal housing sections 121, 122 can be moved closer together to reduce the distance D and thereby increase the height H of the electrode.

In the contracted configuration of FIG. 2, the first catheter 100 has a smaller radial extent and a lower profile. This means that the first catheter 100 can be more easily introduced into and advanced through a blood vessel. If a sheath is used to introduce the first catheter 100 into a vessel, the contracted configuration allows the first catheter 100 to more easily fit into the sheath.

The first catheter 100 may further comprise a catheter sleeve 150 which is disposed around the proximal and distal housing sections 121, 122 and may extend along the length of the catheter shaft 110. The catheter sleeve 150 is a flexible connection element for connecting the proximal and distal housing sections 121, 122 and provides stability to the split housing 120. The catheter sleeve comprises an opening which aligns with opening 123 of the housing to allow the electrode 130 to extend out of the housing 120. The catheter sleeve 150 may be flexible to allow the proximal and distal housing sections 121, 122 to move relative to each other. Alternatively, the proximal housing section 121 or the distal housing section 122 may be slidably moveable within the catheter sleeve 150.

Similar to the catheter 10, the first catheter 100 comprises a proximal set of magnets 141 and a distal set of magnets 142 for aligning the electrode 130 of the first catheter 100 with the backstop 230 of the second catheter 200.

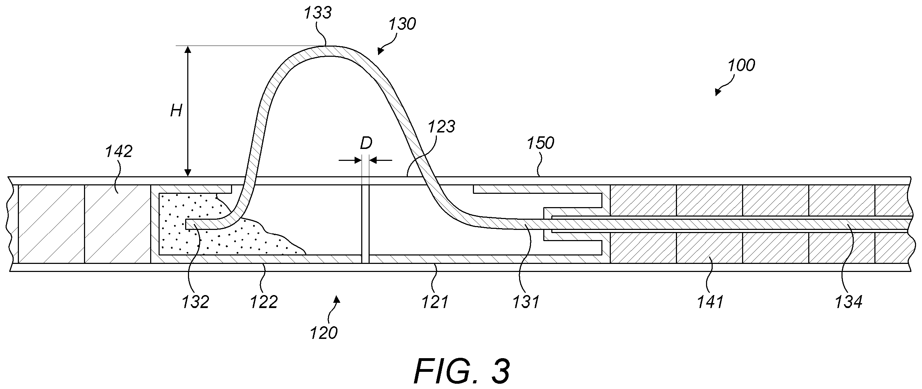

FIG. 3 shows a cross-sectional side view of the first catheter 100 with the electrode 130 in the expanded configuration where the electrode 130 has a greater height H than in the contracted configuration.

In order to move the electrode 130 from the contracted configuration to the expanded configuration, a user can push the proximal housing section 121 in a distal direction. This can be done while the first catheter 100 is inside a vessel. The position of the distal section of the housing may be fixed, for example, via the set of distal magnets 142 which are aligned with a set of magnets of the second catheter 200.

When the user pushes the proximal housing section 121 distally, this causes the distance D between the proximal and distal housing sections 121, 122 to be reduced and thereby increases the height H of the electrode 130. FIG. 3 shows the distance D between the two housing sections in the expanded configuration to be smaller than in the contracted configuration but non-zero, such that the two housing sections do not contact each other. However, the first catheter 100 may also be configured to have the proximal and distal housing sections 121, 122 touching in the expanded configuration. A handle may be connected at the proximal end of the first catheter shaft 110 and assist a user in pushing the proximal housing section 121 distally to increase the electrode height H.

The increased electrode height H allows the electrode 130 to more effectively cut through calcified vessels to form a fistula.

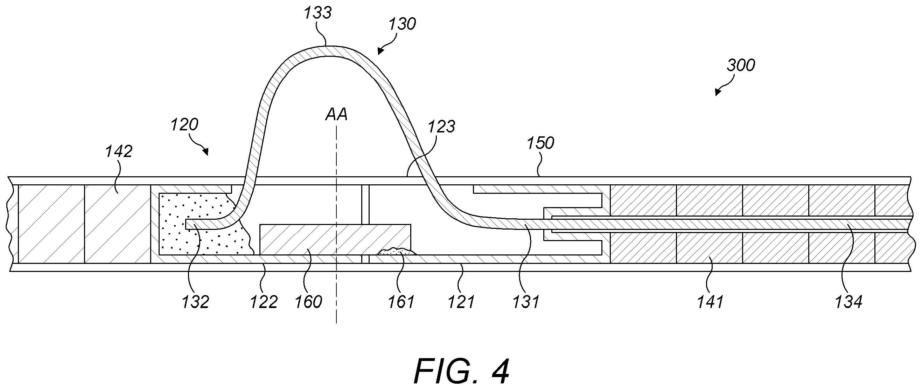

FIG. 4 shows a side view of an alternative embodiment of a first catheter 300 with an electrode 130 in the expanded configuration. The first catheter 300 is similar to the first catheter 100 and the same reference numerals are used to denote identical features.

The first catheter 300 differs from the first catheter 100 in that it further comprises a flexible connection element 160 which may be in the form of a ribbon, a wire or a microspring and may be made from nitinol, for example. The microspring may be a small coil spring or a small leaf spring. The flexible connection element 160 is attached to the proximal housing section 121 using an adhesive 161, for example, and is free to move with respect to the distal housing section 122.

The flexible connection element 160 can be used in addition to, or as an alternative to, the catheter sleeve 150 in order to stabilise the proximal and distal housing sections 121, 122 and maintain their longitudinal alignment. The flexible connection element 160 may be contact with the inner walls of the distal housing section 122 to minimise lateral movement of the distal housing section 122 relative to the proximal housing section 121, whilst still allowing longitudinal movement of the two housing sections relative to each other. The flexible connection element 160 therefore acts as a guide rail for the distal housing section 122. The flexible connection element 160 may be insulated from the electrode 130 as the electrode 130 carries a high current during the fistula forming process. This may prevent arcing between the electrode 130 and the flexible connection element 160.

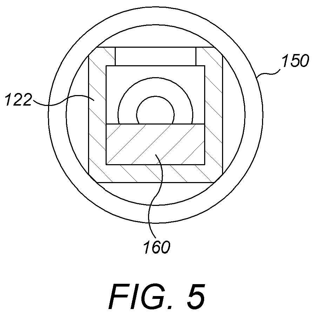

FIG. 5 shows a cross sectional view at line AA of the first catheter 300 of FIG. 4.

As shown in FIG. 5, the catheter sleeve 150 surrounds the distal section of the housing 122. The flexible connection element 160 is positioned inside the housing 122 and contacts the inner walls of the distal housing section 122 to act as a guide rail for the distal housing section 122.

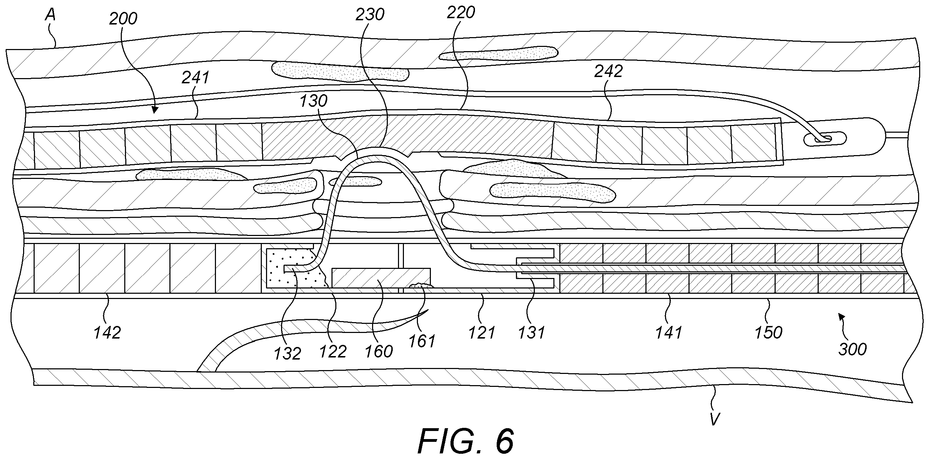

FIG. 6 shows the first catheter 300 and the second catheter 200 in the process of forming an arteriovenous fistula in a calcified vessel. The second catheter 200 may be the same as described with respect to FIG. 1 above. FIG. 6 shows first catheter 300 but this method can equally be performed with first catheter 100.

Firstly, the first catheter 300 is introduced into a vein V through an access site. The second catheter 200 is introduced into an artery A through a second access site.

The first catheter 300 is then advanced through the vein V to the treatment site where the fistula is to be formed. Similarly, the second catheter 200 is advanced through the artery to the treatment site where the fistula is to be formed. The first catheter 300 and/or the second catheter 200 may be advanced to the treatment side inside a sheath. The first catheter 300 and second catheter 200 may be advanced to the treatment site from opposite directions, as shown in FIG. 6, or the same direction.

Once the first catheter 300 and the second catheter 200 are positioned at the treatment site, the proximal set of magnets 141 of the first catheter 300 will be attracted to the distal set of magnets 242 of the second catheter 200 and align themselves with each other. Similarly, the distal set of magnets 141 of the first catheter 300 will be attracted to the proximal set of magnets 241 of the second catheter 200 and these sets of magnets will align with each other. This will result in the electrode 130 becoming aligned with the backstop 230 which is shown with a complementary shaped concave portion. The sets of magnets may also have the effect of pulling the artery A and vein V closer together.

The backstop is configured to compress the tissue in a localised region for ablation by the electrode 130 of the first catheter 300. In order to increase the electrode height H and thereby increase the compression of the tissue between the electrode 130 and the backstop 230, the user may then push the proximal housing section 121 in a distal direction. This can be done by the user through the handle which is connected at the proximal end of the catheter shaft 110.

Since the position of the distal housing section 122 is fixed through alignment of the distal magnets 142 with the magnets 241 of the second catheter 200, distal movement of the proximal housing 121 will reduce the distance D between the two housing sections and result in an increase in height H of the electrode 130. The electrode 130 is thereby moved from the contracted configuration to the expanded configuration.

A radiofrequency (RF) current may then be supplied to the electrode 130 which causes the electrode 130 to heat up generate a plasma. The plasma causes rapid dissociation of molecular bonds in the organic compounds and allows the electrode 130 to cut through the venous and arterial vessel walls until it hits the backstop 230 to form the fistula.

The increased height of the electrode 130 allows the electrode 130 to more easily to cut through thicker, calcified vessel walls.

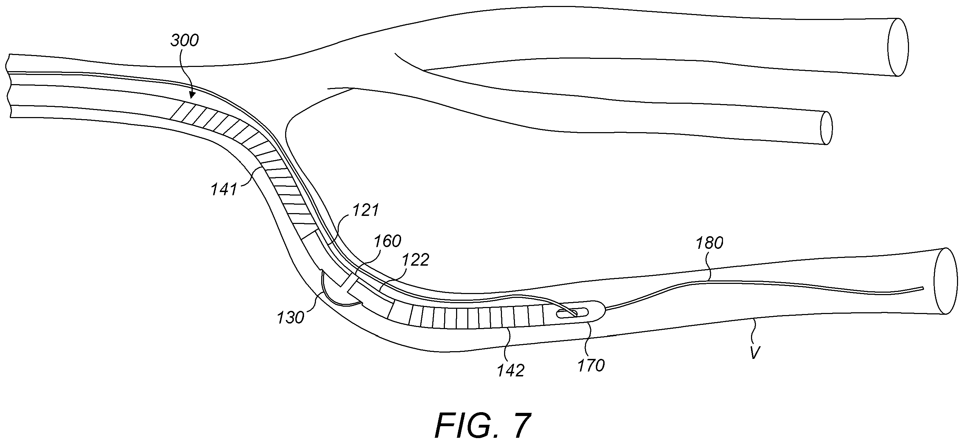

FIG. 7 shows the first catheter 300 in a venous system while it is being delivered to a treatment site. The first catheter 300 may comprise a rapid exchange tip 170 for following a guidewire 180 through the vessel to the treatment site.

Due to the housing 120 being split into a proximal section 121 and a distal section 122, the housing 120 has a degree of flexibility which allows it to more easily navigate arounds bends in the vessel anatomy. The catheter sleeve 150 and the flexible connection element 160 provide stability to the housing 120 while allowing flexing of the proximal and distal sections 121, 122 relative to each other.

The first catheter 100, 300 can therefore more easily navigate tortuous vessel anatomy and be more easily delivered to the treatment site, while still having a housing made from a non-conductive ceramic material to withstand the heat and plasma generated by of the electrode.

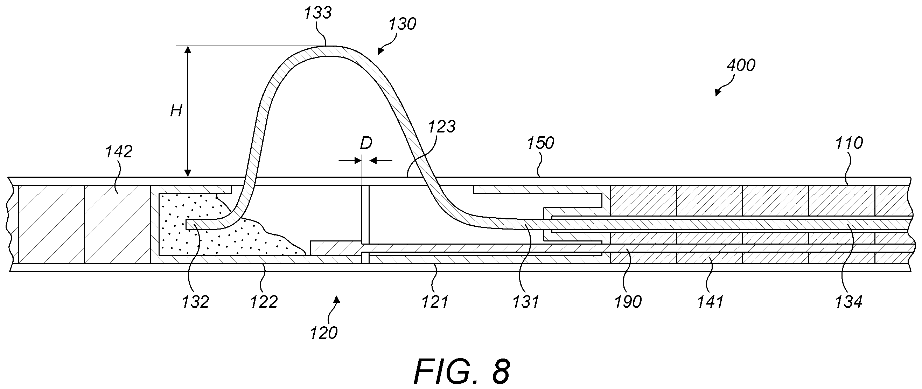

FIG. 8 shows a further alternative embodiment of a first catheter 400. This first catheter is identical to first catheter 100, except that it further comprises a pull wire 190.

The pull wire 190 allows for an alternative means of changing the height H of the electrode 130. For example, the pull wire may be attached to the distal housing section 122 and extend along the catheter shaft 110. By pulling the pull wire, a user can reduce the distance D between the proximal and distal housing sections 121, 122 and thereby increase the height H of the electrode 130.

Various modifications will be apparent to those skilled in the art.

The catheter 300 may also be provided with a pull wire 190. The first catheter 100, 300 may not comprise any proximal magnets 141 or distal magnets 142.

The first catheter 100, 300 may not comprise a flexible connection element 160 in the form of a ribbon, wire or a microspring.

The shape of the electrode 130 is not limited to a convex shape, but could be any other suitable shape, such as for example V-shaped or rectangular-shaped.

The electrode 130 is not limited to a ribbon wire, but may be any other type of suitable wire, for example, a cylindrical wire or oval wire.

The backstop 230 of the second catheter 220 is not limited to a concave shape but may be any suitable shape. For example, the backstop 230 may be recessed or protruding and could have a concave, convex or rectangular shape.

The housing 120 of the first catheter 100, 300 is not limited to a ceramic material and may be made from any suitable material which can withstand the heat and plasma generated by the electrode 130.

The flexible connection element 160 may not touch the inner walls of the distal housing portion 122, but may simply move freely within the housing 122, for example.

The distal portion 132 of the electrode 130 may not be fixed to the distal housing portion 122, but may be allowed some room for movement while still allowing the electrode 130 to move between the contracted and expanded configuration.

The proximal and distal housing section 121, 122 may be in contact or they may not be in contact in the expanded configuration.

## Claims

1. A system for forming a fistula between two vessels, the system comprising a first catheter (100), the first catheter comprising:
a housing (120) having a proximal section (121) and a distal section (122);
an electrode (130) disposed at least partially within the housing (120), the electrode (130) comprising a distal portion (132) connected to the distal section (122) of the housing (120), a proximal portion (131) connected to the proximal section (121) of the housing (120) and an intermediate portion (133) therebetween for contacting a vessel wall and forming the fistula;
wherein the proximal housing section (121) and the distal housing section (122) are longitudinally moveable with respect to each other to move the electrode (130) between a contracted configuration and an expanded configuration, wherein the proximal housing section (121) and the distal housing section (122) are connected via at least one flexible connection element, and
**characterised in that** the at least one flexible connection element comprises a catheter sleeve (150).

2. The system of claim 1, wherein the catheter sleeve (150) is disposed around at least the proximal and distal sections (131, 132) of the housing.

3. The system of any of claims 1 or 2, wherein the at least one flexible connection element comprises a ribbon, wire or microspring (160), optionally wherein the ribbon, wire or microspring (160) is insulated.

4. The system of claim 3, wherein the ribbon, wire or microspring (160) is fixed to one of the housing sections (121, 122) and longitudinally moveable with respect to the other housing section (122, 121).

5. The system of any of claims 3 or 4, wherein the ribbon, wire or microspring (160) is fixed to the proximal housing section (121) and longitudinally moveable with respect to the distal housing section (122), optionally
wherein the ribbon, wire or microspring (160) is in contact with the inner walls of the distal housing section (122).

6. The system of any preceding claim, wherein a pull wire (190) is connected to the distal housing section to allow the distal housing section (122) to be moved longitudinally with respect to the proximal housing section (121), and/or wherein the proximal housing section (121) and the distal housing section (122) are not in contact in the contracted configuration.

7. The system of any preceding claim, wherein the electrode (130) is a leaf spring or a ribbon wire.

8. The system of any preceding claim, wherein the electrode (130) has a convex portion.

9. The system of any preceding claim, wherein a distal end of the electrode (130) is fixed to the distal section of the housing (120), and/or
wherein the housing (120) is at least partially made from a ceramic material.

10. The system of any preceding claim, further comprising a second catheter (200) comprising a second housing (220) and a backstop (230) for the electrode (130).

11. The system of claim 10, wherein the first catheter (100) and the second catheter (200) comprise one or more sets of magnets (141, 142, 241, 242) positioned to align the electrode (130) with the backstop (230).

12. The system of claim 10 or 11, further comprising a distal set of magnets (242) disposed on the second catheter (200), distally of the second housing (220), and/or
further comprising a proximal set of magnets (241) disposed on the second catheter (200), proximally of the second housing (220).

13. The system of any preceding claim, further comprising a distal set of magnets (142) disposed on the first catheter (100), distally of the first housing (120), and/or
further comprising a proximal set of magnets (141) disposed on the first catheter (100), proximally of the first housing (100).

14. The system of any of claims 10 to 13, wherein the backstop (230) is a recessed backstop which has a portion shaped complementary to the electrode (130), and/or
wherein the backstop (230) has a concave portion, and/or
wherein the backstop (230) is non-conductive, and/or
wherein the backstop (230) is at least partly made from a ceramic material.

15. The system of any preceding claim, further comprising a first handle connected to the proximal end of the first catheter (100), and/or
further comprising a radiofrequency generator for supplying radiofrequency energy to the electrode (130).

## Patentansprüche

1. System zur Bildung einer Fistel zwischen zwei Gefäßen, wobei das System einen ersten Katheter (100) umfasst, wobei der erste Katheter Folgendes umfasst:
ein Gehäuse (120), das einen proximalen Abschnitt (121) und einen distalen Abschnitt (122) aufweist;
eine Elektrode (130), die mindestens teilweise innerhalb des Gehäuses (120) angeordnet ist, wobei die Elektrode (130) einen distalen Abschnitt (132), der mit dem distalen Abschnitt (122) des Gehäuses (120) verbunden ist, einen proximalen Abschnitt (131), der mit dem proximalen Abschnitt (121) des Gehäuses (120) verbunden ist, und einen Zwischenabschnitt (133) dazwischen zum Kontaktieren einer Gefäßwand und zum Bilden der Fistel umfasst;
wobei der proximale Gehäuseabschnitt (121) und der distale Gehäuseabschnitt (122) in Längsrichtung relativ zueinander beweglich sind, um die Elektrode (130) zwischen einer zusammengezogenen Konfiguration und einer expandierten Konfiguration zu bewegen, wobei der proximale Gehäuseabschnitt (121) und der distale Gehäuseabschnitt (122) über mindestens ein flexibles Verbindungselement verbunden sind, und
**dadurch gekennzeichnet, dass**
das mindestens eine flexible Verbindungselement eine Katheterhülse (150) umfasst.

2. System nach Anspruch 1, wobei die Katheterhülse (150) mindestens um den proximalen und distalen Abschnitt (131, 132) des Gehäuses angeordnet ist.

3. System nach einem der Ansprüche 1 oder 2, wobei das mindestens eine flexible Verbindungselement ein Band, einen Draht oder eine Mikrofeder (160) umfasst, gegebenenfalls wobei das Band, der Draht oder die Mikrofeder (160) isoliert ist.

4. System nach Anspruch 3, wobei das Band, der Draht oder die Mikrofeder (160) an einem der Gehäuseabschnitte (121, 122) befestigt und in Bezug auf den anderen Gehäuseabschnitt (122, 121) in Längsrichtung beweglich ist.

5. System nach einem der Ansprüche 3 oder 4, wobei das Band, der Draht oder die Mikrofeder (160) an dem proximalen Gehäuseabschnitt (121) befestigt und in Bezug auf den distalen Gehäuseabschnitt (122) in Längsrichtung beweglich ist, gegebenenfalls
wobei das Band, der Draht oder die Mikrofeder (160) in Kontakt mit den Innenwänden des distalen Gehäuseabschnitts (122) ist.

6. System nach einem der vorstehenden Ansprüche, wobei ein Zugdraht (190) mit dem distalen Gehäuseabschnitt verbunden ist, damit der distale Gehäuseabschnitt (122) in Bezug auf den proximalen Gehäuseabschnitt (121) in Längsrichtung bewegt werden kann, und/oder wobei der proximale Gehäuseabschnitt (121) und der distale Gehäuseabschnitt (122) in der zusammengezogenen Konfiguration nicht in Kontakt sind.

7. System nach einem der vorstehenden Ansprüche, wobei die Elektrode (130) eine Blattfeder oder ein Banddraht ist.

8. System nach einem der vorstehenden Ansprüche, wobei die Elektrode (130) einen konvexen Abschnitt aufweist.

9. System nach einem der vorstehenden Ansprüche, wobei ein distales Ende der Elektrode (130) an dem distalen Gehäuseabschnitt (120) befestigt ist, und/oder
wobei das Gehäuse (120) mindestens teilweise aus einem keramischen Material hergestellt ist.

10. System nach einem der vorstehenden Ansprüche, weiter umfassend einen zweiten Katheter (200), der ein zweites Gehäuse (220) und eine Rücklaufsperre (230) für die Elektrode (130) umfasst.

11. System nach Anspruch 10, wobei der erste Katheter (100)
und der zweite Katheter (200) einen oder mehrere Sätze von Magneten (141, 142, 241, 242) umfassen, die so angeordnet sind,
dass sie die Elektrode (130) mit der Rücklaufsperre (230) ausrichten.

12. System nach Anspruch 10 oder 11, weiter umfassend einen distalen Satz von Magneten (242), der an dem zweiten Katheter (200) distal von dem zweiten Gehäuse (220) angeordnet ist, und/oder weiter umfassend einen proximalen Satz von Magneten (241), der an dem zweiten Katheter (200) proximal von dem zweiten Gehäuse (220) angeordnet ist.

13. System nach einem der vorstehenden Ansprüche, weiter umfassend einen distalen Satz von Magneten (142), der auf dem ersten Katheter (100) distal vom ersten Gehäuse (120) angeordnet ist, und/oder weiter umfassend einen proximalen Satz von Magneten (141), der auf dem ersten Katheter (100) proximal des ersten Gehäuses (100) angeordnet ist.

14. System nach einem der Ansprüche 10 bis 13, wobei die Rücklaufsperre (230) eine ausgesparte Rücklaufsperre ist, die einen komplementär zur Elektrode (130) geformten Abschnitt aufweist, und/oder wobei die Rücklaufsperre (230) einen konkaven Abschnitt aufweist, und/oder wobei die Rücklaufsperre (230) nicht leitend ist, und/oder wobei die Rücklaufsperre (230) mindestens teilweise aus einem keramischen Material hergestellt ist.

15. System nach einem der vorstehenden Ansprüche, weiter umfassend einen ersten Griff, der mit dem proximalen Ende des ersten Katheters (100) verbunden ist, und/oder
weiter umfassend einen Radiofrequenzgenerator zur Versorgung der Elektrode (130) mit Radiofrequenzenergie.

## Revendications

1. Système destiné à former une fistule entre deux vaisseaux, le système comprenant un premier cathéter (100), le premier cathéter comprenant :
un boîtier (120) présentant une section proximale (121) et une section distale (122) ;
une électrode (130) disposée au moins partiellement dans le boîtier (120), l'électrode (130) comprenant une partie distale (132) reliée à la section distale (122) du boîtier (120), une partie proximale (131) reliée à la section proximale (121) du boîtier (120) et une partie intermédiaire (133) entre celles-ci pour être en contact avec une paroi de vaisseau et former la fistule ;
dans lequel la section proximale (121) de boîtier et la section distale (122) de boîtier sont longitudinalement mobiles l'une par rapport à l'autre pour déplacer l'électrode (130) entre une configuration contractée et une configuration déployée, dans lequel la section proximale (121) de boîtier et la section distale (122) de boîtier sont reliés via au moins un élément de liaison flexible, et
**caractérisé en ce que**
le au moins un élément de liaison flexible comprend un manchon (150) de cathéter.

2. Système selon la revendication 1, dans lequel le manchon (150) de cathéter est disposé autour d'au moins les sections proximale et distale (131, 132) du boîtier.

3. Système selon l'une quelconque des revendications 1 ou 2, dans lequel le au moins un élément de liaison flexible comprend un ruban, fil ou micro-ressort (160), facultativement dans lequel le ruban, fil ou micro-ressort (160) est isolé.

4. Système selon la revendication 3, dans lequel le ruban, fil ou micro-ressort (160) est fixé à l'une des sections (121, 122) de boîtier et longitudinalement mobile par rapport à l'autre section (122, 121) de boîtier.

5. Système selon l'une quelconque des revendications 3 ou 4, dans lequel le ruban, fil ou micro-ressort (160) est fixé à la section proximale (121) de boîtier et longitudinalement mobile par rapport à la section distale (122) de boîtier, facultativement
dans lequel le ruban, fil ou micro-ressort (160) est en contact avec les parois internes de la section distale (122) de boîtier.

6. Système selon une quelconque revendication précédente, dans lequel un fil de traction (190) est relié à la section distale de boîtier pour permettre à la section distale (122) de boîtier d'être longitudinalement déplacée par rapport à la section proximale (121) de boîtier, et/ou dans lequel la section proximale (121) de boîtier et la section distale (122) de boîtier ne sont pas en contact dans la configuration contractée.

7. Système selon une quelconque revendication précédente, dans lequel l'électrode (130) est un ressort à lames ou un fil méplat.

8. Système selon une quelconque revendication précédente, dans lequel l'électrode (130) présente une partie convexe.

9. Système selon une quelconque revendication précédente, dans lequel une extrémité distale de l'électrode (130) est fixée à la section distale du boîtier (120), et/ou
dans lequel le boîtier (120) est au moins partiellement créé à partir d'un matériau céramique.

10. Système selon une quelconque revendication précédente, comprenant en outre un second cathéter (200) comprenant un second boîtier (220) et un appui (230) pour l'électrode (130).

11. Système selon la revendication 10, dans lequel le premier cathéter (100) et le second cathéter (200) comprennent un ou plusieurs ensembles d'aimants (141, 142, 241, 242) positionnés pour aligner l'électrode (130) avec l'appui (230).

12. Système selon la revendication 10 ou revendication 11, comprenant en outre un ensemble distal d'aimants (242) disposés sur le second cathéter (200), de manière distale au second boîtier (220), et/ou comprenant en outre un ensemble proximal d'aimants (241) disposés sur le second cathéter (200), de manière proximale au second boîtier (220).

13. Système selon une quelconque revendication précédente, comprenant en outre un ensemble distal d'aimants (142) disposés sur le premier cathéter (100), de manière distale au premier boîtier (120), et/ou
comprenant en outre un ensemble proximal d'aimants (141) disposés sur le premier cathéter (100), de manière proximale au premier boîtier (100).

14. Système selon l'une des revendications 10 à 13, dans lequel l'appui (230) est un appui enfoncé qui présente une partie de forme complémentaire à celle de l'électrode (130), et/ou
dans lequel l'appui (230) présente une partie concave, et/ou
dans lequel l'appui (230) est non conducteur, et/ou
dans lequel l'appui (230) est au moins partiellement créé à partir d'un matériau céramique.

15. Système selon une quelconque revendication précédente, comprenant en outre une première poignée reliée à l'extrémité proximale du premier cathéter (100), et/ou
comprenant en outre un générateur de radiofréquence destiné à apporter de l'énergie de radiofréquence à l'électrode (130).
